(19) Europäisches Patentamt
European Patent Office
Office européen des brevets

(11) Veröffentlichungsnummer: **0 081 128**
**A1**

(12) # EUROPÄISCHE PATENTANMELDUNG

(21) Anmeldenummer: **82110733.1**

(22) Anmeldetag: **20.11.82**

(51) Int. Cl.³: **C 07 C 79/46**
C 07 C 67/313
//C07C69/712

(30) Priorität: **03.12.81 DE 3147884**

(43) Veröffentlichungstag der Anmeldung:
**15.06.83 Patentblatt 83/24**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Priesnitz, Uwe, Dr.**
**Severinstrasse 58**
**D-5650 Solingen 1(DE)**

(54) **Verfahren zur Herstellung von alpha-(5-(4-Trifluormethyl-phenoxy)-2-nitro-phenoxy)-propionsäure-(alkoxy-carbonyl-methyl)-estern.**

(57) Verfahren zur Herstellung von bekannten α-[5-(4-Trifluormethyl-phenoxy)-2-nitro-phenoxy]-propionsäure-(alkoxycarbonyl-methyl)-estern der Formel

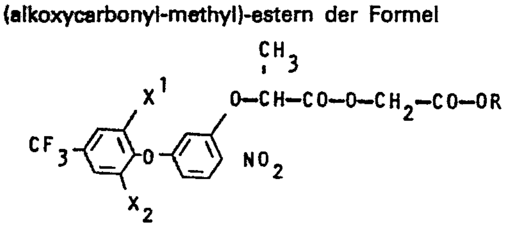

(I)

in welcher
$X^1$ und $X^2$ unabhängig von einander für Wasserstoff oder Halogenstehen und
R für gegebenenfalls substituiertes Alkyl steht,
durch Umsetzung von α-[3-(4-Trifluor-methyl-phenoxy)-phenoxy]-propionsäure-(alkoxycarbonyl-methyl)-estern der Formel

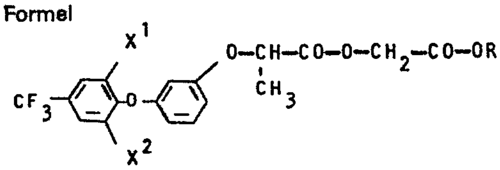

(II)

in welcher
$X^1$, $X^2$ und R die oben angegebene Bedeutung haben, mit Übergangsmetallnitraten in Gegenwart von Essigsäureanhydrid bei Temperaturen zwischen −20 und +80°C.

EP 0 081 128 A1

BAYER AKTIENGESELLSCHAFT 5090 Leverkusen, Bayerwerk
Zentralbereich
Patente, Marken und Lizenzen Dü/Fr
IVa

Verfahren zur Herstellung von α-[5-(4-Trifluormethyl-phenoxy)-2-nitro-phenoxy]-propionsäure-(alkoxy-carbonyl-methyl)-estern

Die Erfindung betrifft ein neues Verfahren zur Herstellung von bekannten α-[5-(4-Trifluormethyl-phenoxy)-2-nitro-phenoxy]-propionsäure-(alkoxy-carbonyl-methyl)-estern, welche herbizide Eigenschaften besitzen.

Es ist bereits bekannt geworden, daß man bestimmte α-[5-(4-Trifluormethyl-phenoxy)-2-nitro-phenoxy]-propionsäure-(alkoxycarbonyl-methyl)-ester erhält, wenn man entsprechende α-[5-(4-Trifluormethyl-phenoxy)-2-nitro-phenoxy]-propionsäurechloride mit Hydroxyessigsäurealkylestern in Gegenwart von Säurebindemitteln umsetzt (vergleiche DE-OS 2 906 087). Nach dieser Verfahrensweise erhält man die gewünschten Produkte jedoch in für praktische Zwecke zu geringen Ausbeuten und unbefriedigender Qualität.

Es wurde nun gefunden, daß man bekannte α-[5-(4-Trifluormethyl-phenoxy)-2-nitro-phenoxy]-propionsäure-(alkoxycarbonyl-methyl)-ester der Formel

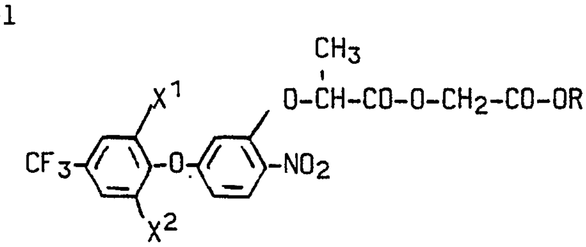

(I)

in welcher

$X^1$ und $X^2$ unabhängig von einander für Wasserstoff oder Halogen stehen und

R für gegebenenfalls substituiertes Alkyl steht,

erhält, wenn man α-[3-(4-Trifluor-methyl-phenoxy)-phenoxy]-propionsäure-(alkoxy-carbonyl-methyl)-ester der Formel

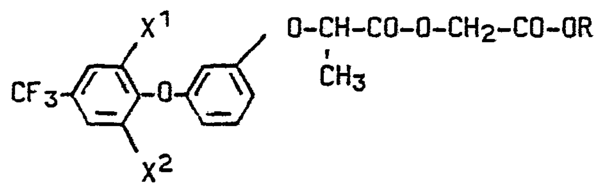

(II)

in welcher

$X^1$, $X^2$ und R die oben angegebene Bedeutung haben,

mit einem Uebergangsmetallnitrat in Gegenwart von Essigsäureanhydrid bei Temperaturen zwischen -2o und +8o°C umsetzt.

Es ist als ausgesprochen überraschend zu bezeichnen, daß man die α-[5-(4-Trifluormethyl-phenoxy)-2-nitro-phenoxy]-propionsäure-(alkoxy-carbonyl-methyl)-ester der Formel (I) nach dem erfindungsgemäßen Verfahren in sehr hohen Ausbeuten und nahezu frei von Verunreinigungen durch isomere Nitrierungsprodukte erhält, da ein so regio-selektiver Reaktionsverlauf im allgemeinen bei der Nitrierung von Aromaten nicht beobachtet wird.

Das erfindungsgemäße Verfahren besitzt eine Reihe von Vorteilen. So sind die als Ausgangsstoffe benötigten Substanzen auch in

größerer Menge in einfacher Weise zugänglich und auch im technischen Maßstab problemlos zu handhaben. Ferner ist der zur Durchführung des erfindungsgemäßen Verfahrens erforderliche apparative Aufwand gering und die Aufarbeitung nach beendeter Umsetzung bereitet keine Schwierigkeiten. Insbesondere ist der bisher nicht erreichte hohe Reinheitsgrad der Produkte der Formel (I), welche man zudem in sehr hoher Ausbeute erhält, hervorzuheben. Das erfindungsgemäße Verfahren stellt somit eine wertvolle Bereicherung der Technik dar.

Verwendet man α-[3-(4-Trifluormethyl-phenoxy)-phenoxy]-propionsäure-(ethoxycarbonyl-methyl)-ester als Ausgangsstoff und Eisen-(III)-nitrat als Nitrierungsmittel, so kann der Verlauf des erfindungsgemäßen Verfahrens durch das folgende Formelschema wiedergegeben werden:

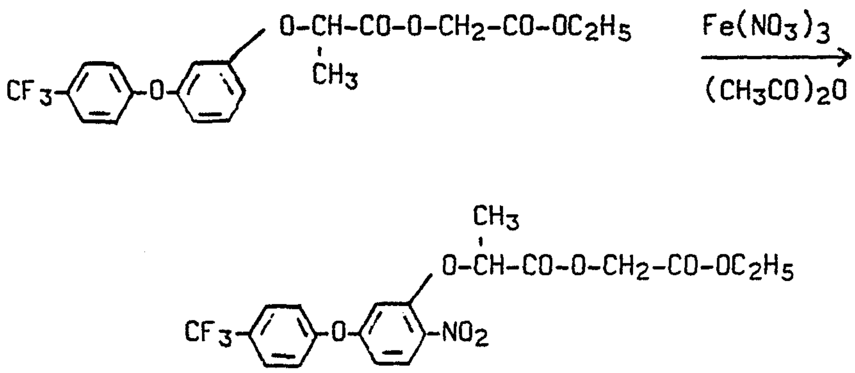


Die beim erfindungsgemäßen Verfahren als Ausgangsstoffe zu verwendenden α-[3-(4-Trifluormethyl-phenoxy)-phenoxy]-propionsäure-(alkoxycarbonyl-methyl)-ester sind durch die Formel (II) allgemein definiert. Vorzugsweise stehen darin

$X^1$ für Wasserstoff oder Chlor,

$X^2$ für Wasserstoff oder Chlor und

R für geradkettiges oder verzweigtes Alkyl mit 1 bis 5 Kohlenstoffatomen.

Als Beispiele für die Verbindungen der Formel (II) seien genannt: α-[3-(2-Chlor-4-trifluormethyl-phenoxy)-phenoxy]-propionsäure-(methoxycarbonyl-methyl)-ester,-(ethoxycarbonyl-methyl-ester, -(n-propoxycarbonyl-methyl)-ester, -(iso-propoxycarbonyl-methyl)-ester und -(n-butoxycarbonyl-methyl)-ester sowie α-[3-(2,6-Dichlor-4-trifluormethyl-phenoxy)-phenoxy7-propionsäure-(methoxycarbonyl-methyl)-ester, -(ethoxycarbonyl-methyl)-ester, -(n-propoxycarbonylmethyl)-ester, -(iso-propoxycarbonyl-methylester)- und -(n-butoxycarbonyl-methyl)-ester.

Verbindungen der Formel (II) sind bereits bekannt oder lassen sich nach bekannten Verfahren in einfacher Weise herstellen (vgl. DE-OS 2 805 981). So erhält man die Verbindungen der Formel (II) zum Beispiel, indem man 3-(4-Trifluormethyl-phenoxy)-phenole der Formel

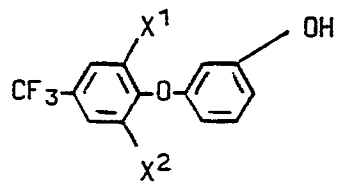

(III)

in welcher

$X^1$ und $X^2$ die oben angegebene Bedeutung haben,

mit α-Brom-propionsäure-(alkoxycarbonyl-methyl)-estern der Formel

$$Br-\underset{|}{\overset{CH_3}{C}}H-CO-O-CH_2-CO-OR \qquad (IV)$$

in welcher

R die oben angegebene Bedeutung hat,

in Gegenwart eines Säurebindemittels, wie z.B. Kaliumcarbonat, und in Gegenwart eines Verdünnungsmittels, wie z.B. Aceton, bei Temperaturen zwischen 4o und 7o°C umsetzt. Die Aufarbeitung wird nach üblichen Methoden durchgeführt.

Die bei der Herstellung der Verbindungen der Formel (II) als Ausgangsprodukte benötigten 3-(4-Trifluormethyl-phenoxy)-phenole der Formel (III) und die α-Brom-propionsäure-(alkoxycarbonyl-methyl)-ester der Formel (IV) sind bekannt (vgl. zum Beispiel DE-OS 2 805 981).

Als Beispiele für Übergangsmetallnitrate, welche beim erfindungsgemäßen Verfahren als Nitrierungsmittel verwendet werden, seien Kupfer-(II)-nitrat, Eisen-(III)-nitrat, Kobalt-(II)-nitrat, Kobalt-(III)-nitrat, Nickel-(II)-nitrat, Chrom-(III)-nitrat und Mangan-(II)-nitrat genannt. Vorzugsweise wird Kupfer-(II)-nitrat oder Eisen-(III)-nitrat eingesetzt.

Die Reaktionstemperaturen können bei dem erfindungsgemäßen Verfahren innerhalb eines bestimmten Bereiches variiert werden. Vorzugsweise arbeitet man bei Temperaturen zwischen 0 und 6o°C, insbesondere zwischen lo und 5o°C.

Das erfindungsgemäße Verfahren wird im allgemeinen bei Normaldruck durchgeführt.

Zur Durchführung des erfindungsgemäßen Verfahrens setzt man auf 1 Mol α-[3-(4-Trifluormethyl-phenoxy)-phenoxy]-propionsäure-(alkoxycarbonyl-methyl)-ester der Formel (II) 1 bis 2 Moläquivalente, vorzugsweise 1,1 bis 1,5 Moläquivalente eines Uebergangsmetallnitrats und 1 bis lo Mol, vorzugsweise 3 bis 8 Mol Essigsäureanhydrid ein. Die Ausgangsstoffe werden im allgemeinen bei Raumtemperatur oder gegebenenfalls bei leicht erhöhter Temperatur zusammengegeben und bis zum Ende der Umsetzung gerührt.

In einer bevorzugten Ausführungsform des erfindungsgemäßen Verfahrens wird das Uebergangsmetallnitrat in Acetanhydrid vorgelegt. Zu dieser Mischung wird eine Lösung der Ausgangsverbindung der Formel (II) in Acetanhydrid tropfenweise gegeben und das Reaktionsgemisch wird bis zum Reaktionsende gerührt.

Die Isolierung der Reaktionsprodukte erfolgt nach üblichen Methoden. Im allgemeinen geht man so vor, daß man das Reaktionsgemisch in Eiswasser gießt, das dabei entstehende Gemisch mit einem mit Wasser praktisch nicht mischbaren organischen Lösungsmittel, wie zum Beispiel Diethylether oder Methylenchlorid, mehrfach extrahiert, die vereinigten organischen Phasen wäscht, trocknet und dann unter vermindertem Druck einengt. Den verbleibenden öligen Rückstand überführt man durch Behandlung mit geeigneten organischen Solventien, wie zum Beispiel Methanol, in den festen Zustand und kristallisiert gegebenenfalls um, zum Beispiel aus einem Alkohol, wie Isopropanol, oder aus Cyclohexan oder auch aus Wasser. Die anfallenden Stoffe werden durch ihre Schmelzpunkte sowie durch ihre Spektren charakterisiert.

Die nach dem erfindungsgemäßen Verfahren herstellbaren α-[5-(4-Trifluormethyl-phenoxy)-2-nitro-phenoxy]-propionsäure-(alkoxycarbonyl-methyl)-ester der Formel (I) besitzen sehr gute herbizide Eigenschaften (vgl. DE-OS 2 906 087).

Das erfindungsgemäße Verfahren und dessen unerwartete Überlegenheit gegenüber dem bisherigen Verfahren zur Herstellung der Verbindungen der Formel (I) werden durch die nachfolgenden Beispiele veranschaulicht.

Vergleichsbeispiele

Beispiel A

Herstellung der Verbindung der Formel

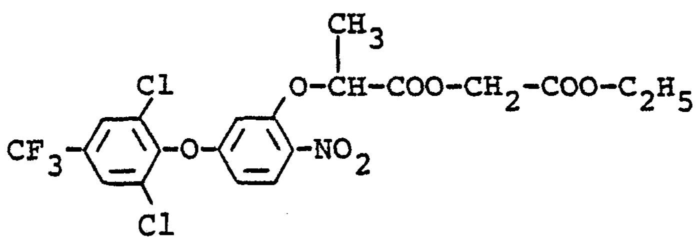

nach dem Verfahren gemäß DE-OS 2 906 087.

Eine Lösung von 15g 5-(2,6-Dichlor-4-trifluormethyl-phenoxy)-2-nitro-$\alpha$-phenoxy-propionsäurechlorid in 30 ml Toluol wird zu einer auf 0 bis 5°C gekühlten Lösung von 3,7g Hydroxy-essigsäure-ethylester und 4g Triethylamin in 70 ml Toluol tropfenweise gegeben. Das Reaktionsgemisch wird über Nacht bei Raumtemperatur gerührt, mit 300 ml Toluol verdünnt, neutral gewaschen, getrocknet, filtriert und eingeengt. Man erhält 13,5g 5-(2,6-Dichlor-4-trifluormethyl-phenoxy)-2-nitro-$\alpha$-phenoxy-propionsäure-(ethoxycarbonyl-methyl)-ester zunächst in Form eines gelbstichigen Öles, das dann kristallisiert.
Schmelzpunkt: 75-76°C.
Die Ausbeute an reiner Substanz errechnet sich zu 70,5 % der Theorie.

Erfindungsgemäße Beispiele:

Beispiel 1

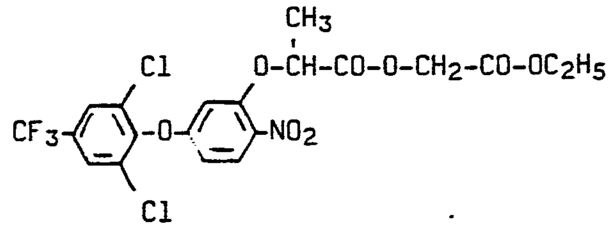

Eine Mischung aus 48 g (o,1 Mol) α-[3-(2,6-Dichlor-4-trifluormethyl-phenoxy)-phenoxy]-propionsäure-(ethoxycarbonyl-methyl)-ester und 2o ml Acetanhydrid wird innerhalb von 30 Minuten tropfenweise und unter Rühren zu einer zuvor auf 30°C erwärmten Mischung aus 13,8 g (0,06 Mol) Kupfer-(II)-nitrat-trihydrat und 47 ml Acetanhydrid gegeben. Das Reaktionsgemisch wird weitere 30 Minuten gerührt und dann in Eiswasser gegossen. Man extrahiert das entstehende Gemisch mehrfach mit Diethylether, wäscht die vereinigten organischen Phasen mit Wasser, trocknet, filtriert und zieht vom Filtrat das Lösungsmittel unter vermindertem Druck sorgfältig ab. Man erhält 53g (100 % der Theorie) eines öligen Produktes, welches nach seinem $^1$H-NMR-Spektrum mehr als 90 % an α-[5-(2,6-Dichlor-4-trifluormethyl-phenoxy)-2-nitro-phenoxy]-propionsäure-(ethoxycarbonyl-methyl)-ester enthält. Durch Behandeln mit Methanol erhält man 30 g ( 58 % der Theorie) reines Produkt vom Schmelzpunkt 77°C.

Herstellung des Ausgangsproduktes der Formel

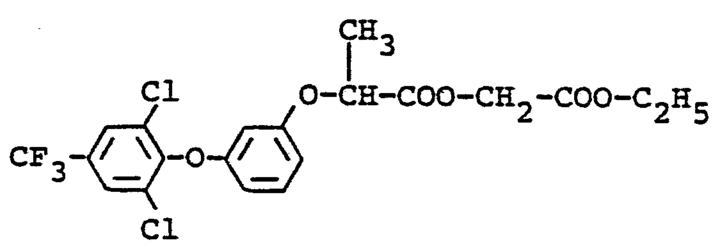

(II-1)

Zu einer Mischung aus 80,8g (0,25 Mol) 3-(2,6-Dichlor-4-trifluormethyl-phenoxy).phenol, 350 ml Aceton und 41,4g (0,3 Mol) Kaliumcarbonat werden unter Rühren bei 50-60°C 63g (0,2625 Mol) α-Brompropionsäure-(ethoxycarbonyl-methyl)-ester getropft. Anschließend wird das Reaktionsgemisch weitere 7 Stunden bei 50-60°C gerührt. Dann wird aufgearbeitet, indem man das Reaktionsgemisch mit 500 ml Wasser und 500 ml Toluol versetzt, die Phasen trennt, die wäßrige Phase einmal mit 200 ml Toluol extrahiert, die vereinigten organischen Phasen einmal mit 200 ml 5%iger wäßriger Natronlauge und einmal mit 200 ml Wasser wäscht und nach dem Trocknen über Natriumsulfat das Lösungsmittel unter vermindertem Druck abzieht. Man erhält auf diese Weise 90,8g (75,5 % der Theorie) an 3-(2,6-Dichlor-4-trifluormethyl-phenoxy)-α-phenoxy-propionsäure-(ethoxy-carbonyl-methyl)-ester in Form eines gelben Öles.

<u>Beispiel 2</u>

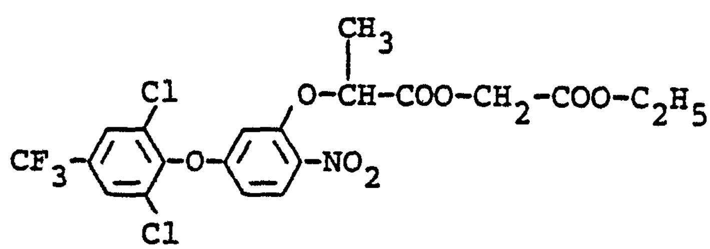

Setzt man bei der Durchführung der im Beispiel 1 beschriebenen Umsetzung an Stelle von Kupfer-(II)-nitrat die gleiche Stoffmenge an Eisen-(III)-nitrat ein und arbeitet man völlig analog zu der im Beispiel 1 angegebenen Weise, so erhält man 52 g (98 % der Theorie) eines öligen Produktes, welches nach seinem $^1$H-NMR-Spektrum mehr als 90 % an α-[5-(2,6-Dichlor-4-trifluormethyl-phenoxy)-2-nitro-phenoxy]-propionsäure-(ethoxy-carbonyl-methyl)-ester enthält.

Patentansprüche

1) Verfahren zur Herstellung von α-[5-(4-Trifluormethyl-phenoxy)-2-nitro-phenoxy]-propionsäure-(alkoxycarbonyl-methyl)-estern der Formel

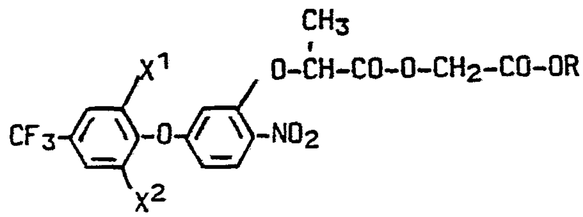

(I)

in welcher

$X^1$ und $X^2$ unabhängig von einander für Wasserstoff oder Halogen stehen und

R für gegebenenfalls substituiertes Alkyl steht,

dadurch gekennzeichnet, daß man α-[3-(4-Trifluor-methyl-phenoxy)-phenoxy]-propionsäure-(alkoxycarbonyl-methyl)-ester der Formel

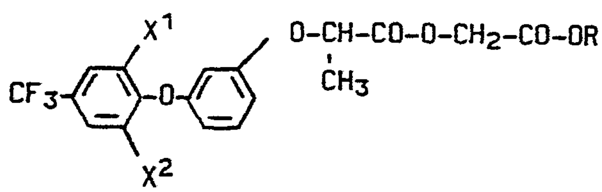

(II)

in welcher

$X^1$, $X^2$ und R die oben angegebene Bedeutung haben,

mit einem Uebergangsmetallnitrat in Gegenwart von Essigsäureanhydrid bei Temperaturen zwischen -2o und +8o°C umsetzt.

2) Verfahren gemäß Anspruch 1, dadurch gekennzeichnet, daß man als Übergangsmetallnitrat Kupfer-(II)-nitrat, Eisen-(III)-nitrat, Cobalt-(II)-nitrat, Cobalt-(III)-nitrat, Nickel-(II)-nitrat, Chrom-(III)-nitrat oder Mangan-(II)-nitrat einsetzt.

3) Verfahren gemäß Anspruch 1, dadurch gekennzeichnet, daß man die Umsetzung bei Temperaturen zwischen 0°C und 60°C durchführt.

4) Verfahren gemäß Anspruch 1, dadurch gekennzeichnet, daß man als Ausgangsverbindung der Formel (II) α-[3-(2,6-Dichlor-4-trifluormethyl-phenoxy)-phenoxy]-propionsäure-(ethoxycarbonyl-methyl)-ester der Formel

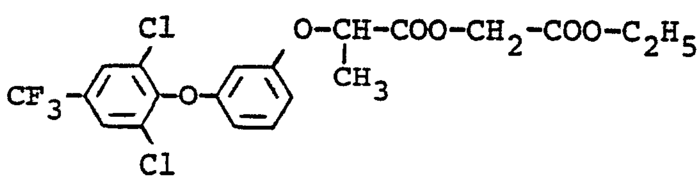

(II-1)

einsetzt.

0081128

Europäisches Patentamt

# EUROPÄISCHER RECHERCHENBERICHT

Nummer der Anmeldung

EP 82 11 0733

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| --- | --- | --- | --- |
| A | HOUBEN-WEYL: "Methoden der organischen Chemie", Band 10/1, Teil 1, Seiten 768-773, Georg Thieme Verlag, Stuttgart, DE. * Seiten 768-773 * ----- | 1-3 | C 07 C 79/46<br>C 07 C 67/313//<br>C 07 C 69/712 |
| | | | RECHERCHIERTE SACHGEBIETE (Int. Cl. 3) |
| | | | C 07 C 69/00<br>C 07 C 67/00<br>C 07 C 79/00 |

Der vorliegende Recherchenbericht wurde für alle Patentansprüche erstellt.

| Recherchenort | Abschlußdatum der Recherche | Prüfer |
| --- | --- | --- |
| DEN HAAG | 07-03-1983 | KINZINGER J.M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82